# EUROPÄISCHE PATENTSCHRIFT

(11) **EP 0 361 253 B1**
(45) Veröffentlichungstag und Bekanntmachung des Hinweises auf die Patenterteilung: **15.12.1993**
(21) Anmeldenummer: 89117200.9
(22) Anmeldetag: 18.09.1989
(51) Int. Cl.: C09B 1/50

(54) **Verfahren zur Herstellung von 1-Amino-2-chlor-4-hydroxy-anthrachinon**
Process for the preparation of 1-amino-2-chloro-4-hydroxy-anthraquinone
Procédé de préparation de la 1-amino-2-chloro-4-hydroxy-anthraquinone

(30) Priorität: 27.09.1988 DE 3832740
(43) Veröffentlichungstag der Anmeldung: 04.04.1990
(73) Patentinhaber: BASF Aktiengesellschaft, 67063 Ludwigshafen (DE)
(72) Erfinder: Epple, Gerhard, Dr., D-6719 Weisenheim (DE)

(56) Entgegenhaltungen:
- DE-A- 1 184 879
- DE-A- 2 428 337

## Beschreibung

Aus der DE-A 2 163 059 ist es bekannt, (I) durch Cyclisierung von 3'-Nitro-4'-chlorbenzoyl-benzoesäure mit Borsäure und Oleum und anschließende Reduktion der Nitrogruppe herzustellen. Dieses Verfahren erfordert jedoch eine aufwendige Reinigung des Produkts, und die Ausbeuten an (I) sind unbefriedigend.

Nach der DE-A-2 428 337 kann man (I) durch Umsetzung der 1-amino-2,4-dichlor-anthrachinons durch Hydroxilierung in Schwefelsäure in Gegenwart von Borsäure erhalten.

Nach der DE-A 1 215 283 kann man (I) durch Oxidation von (II) mit Mangandioxid zum entsprechenden Chinonimin und anschließender Anlagerung von Chlorwasserstoff herzustellen.

In der JP-B 42631/1981 wird die Herstellung von (I) durch Chlorierung der mineralsauren Salze von (II) in inerten organischen Lösungsmitteln beschrieben. Weiterhin ist aus der DE-B 1 199 279 bekannt, daß man eine Chlorierung in der 5- und der 8-Position von (II) in schwefelsaurem Medium in Gegenwart von Borsäure durchführen kann. Die selektive Chlorierung der 2-Position bereitet jedoch Schwierigkeiten, denn meist werden dichlorierte Produkte gebildet.

Da die bisher bekannten Verfahren in Bezug auf Reinheit und Ausbeuten unbefriedigend waren, lag der Erfindung die Aufgabe zugrunde, diesen Mängeln abzuhelfen.

Diese Aufgabe wird mit Hilfe des Verfahrens der vorliegenden Erfindung gelöst.

Die Erfindung betrifft ein Verfahren zur Herstellung von 1-Amino-2-chlor-4-hydroxy-anthrachinon (I) durch Chlorierung von 1-Amino-4-hydroxy-anthrachinon (II), welches dadurch gekennzeichnet ist, daß man die Chlorierung in 90 bis 100 gew.-%iger Schwefelsäure oder in bis zu 2 gew.-%igem Oleum durchführt.

1-Amino-4-hydroxy-anthrachinon (II) ist bekannt und nach bekannten Verfahren erhältlich, z.B. durch Umsetzung von 1,4-Diamino-anthrachinon mit Mangandioxid in Schwefelsäure. Für die meisten Zwecke genügt es, (II) in technischer Qualität (Reingehalt 95 Gew.-%) einzusetzen.

Die Schwefelsäure bzw. das Oleum dient als Lösungsmittel für (II), und zwar vorzugsweise in Mengen von 5 bis 20, insbesondere 6 bis 12 kg pro kg (II). Die Konzentration der Schwefelsäure beträgt 90 bis 100, vorzugsweise 92 bis 98 Gew.-%. Der zu 100 % verbleibende Rest ist Wasser.

Auch Oleum, also eine Lösung von Schwefeltrioxid in wasserfreier Schwefelsäure, ist geeignet, wobei die SO₃-Konzentration bis zu 2 Gew.-% betragen kann.

Als Chlorierungsmittel verwendet man vorzugsweise Chlor, jedoch kommen auch das Natriumsalz der Dichlorisocyanursäure und Chlorkalk in Betracht.

Verwendet man Chlor als Chlorierungsmittel, läßt sich die Reaktion durch Mitverwendung eines Chlorierungskatalysators wie Eisen-III-chlorid oder Mangandioxid beschleunigen.

Chlorierungsmittel und (II) werden vorzugsweise im stöchiometrischen Verhältnis eingesetzt, jedoch kann es sich zur Beschleunigung der Reaktion auch empfehlen, das Chlorierungsmittel in einem Überschuß von bis zu etwa 20 mol.äq.-% zu verwenden. Ein stöchiometrischer Überschuß von (II), etwa um die Selektivität von (I) zu erhöhen, ist im Hinblick auf die Aufarbeitung des Reaktionsgemisches hingegen weniger ratsam.

Im allgemeinen nimmt man die Chlorierung bei 20 bis 100, vorzugsweise bei 40 bis 75°C vor.

Verfahrenstechnisch geht man im allgemeinen so vor, daß man eine Lösung von (II) in Schwefelsäure bzw. Oleum vorlegt, hierzu bei der Reaktionstemperatur das Chlorierungsmittel gibt und die Temperatur gegebenenfalls im Laufe der fortschreitenden Reaktion erhöht.

Man kann die Umsetzung nach den üblichen Techniken auch kontinuierlich, z.B. in einer Rührkesselkaskade, vornehmen.

Der Reaktionsverlauf wird zweckmäßigerweise dünnschichtchromatographisch überwacht. Wenn mehr als 97 % von (II) umgesetzt sind, bricht man die Reaktion im allgemeinen ab, indem man das Reaktionsgemisch entweder auf Wasser oder eine wäßrige Natriumhydrogensulfitlösung gibt, oder das Reaktionsgemisch langsam mit Wasser verdünnt.

Der hierbei ausfallende Feststoff wird abfiltriert und mit Wasser gewaschen. Er enthält in der Regel 90 bis 95 Gew.-% (I). Die Restmenge besteht hauptsächlich aus nicht umgesetztem (II), wogegen der Anteil der unerwünschten dichlorierten Produkte praktisch vernachlässigt werden kann.

1-Amino-2-chlor-4-hydroxyanthrachinon (I) ist bekannterweise ein wichtiges Zwischenprodukt bei der Herstellung von Antrachinonfarbstoffen, z.B. von Disperse Red 60 und Disperse Red 91.

### Beispiel 1

Eine bei 20 bis 25°C hergestellte Lösung von 158 g 1-Amino-4-hydroxy-anthrachinon (95 gew.-%ig) in 1500 g 96 gew.%iger Schwefelsäure wurde bei 55 bis 60°C mit 3 g wasserfreiem Eisen-III-chlord versetzt. Anschließend wurde so lange Chlor durchgeleitet, bis die Ausgangsverbindung (II) dünnschichtchromatographisch nur noch in Spuren nachweisbar war. Dann wurde das Reaktionsgemisch zu einer Lösung aus 45 g Natriumhydrogensulfit und 5000 g Wasser gegeben. Das ausgefallende Reaktionsprodukt wurde abfiltriert und mit Wasser gewaschen.

Man erhielt 174 g Produkt mit einem Gehalt von 93 Gew.-% an (I), was einer Ausbeute von 94 % d. Th. entspricht.

### Beispiel 2

Eine bei 20 bis 25°C hergestellte Lösung von 30 g 1-Amino-4-hydroxy-anthrachinon (95 gew.-%ig) in 300 g 96 gew.-%iger Schwefelsäure wurde bei 60°C während einer Zeitspanne von 15 min. mit 16 g Na-Salz der Dichlorisocyanursäure versetzt. Anschließend wurde das Gemisch noch 1 Stunde lang bei 60°C gerührt und dann zu 1000 g Wasser gegeben. Das Reaktionsprodukt wurde analog Beispiel 1 aufgearbeitet.

Man erhielt 34 g Produkt mit einem Gehalt an (I) von 89 Gew.-% was einer Ausbeute von 93 % der Theorie entspricht.

### Beispiel 3

Eine Lösung von 50 g 1-Amino-4-hydroxy-anthrachinon (94,5 gew.-%ig) in 300 g 96 gew.-%iger Schwefelsäure wurde mit 2 g wasserfreiem Eisen-III-chlorid versetzt. Bei 60°C wurde so lange Chlor eingeleitet bis die Ausgangsverbindung (II) dünnschichtchromatographisch nur noch in Spuren nachweisbar war. Dann wurden 2 g Natriumhydrogensulfit zugegeben und bei 60 bis 80°C 84 g Wasser zugetropft. Nach dem Abkühlen auf Raumtemperatur wurde das ausgefallene Reaktionsprodukt abgesaugt, mit 250 g 60 gew.-%iger wäßriger Schwefelsäure und anschließend mit Wasser gewaschen.

Man erhielt 54,6 g Produkt mit einem Gehalt an (I) von 95,2 Gew.-%, was einer Ausbeute von 96 % der Theorie entspricht.

## Patentansprüche

1. Verfahren zur Herstellung von 1-Amino-2-chlor-4-hydroxy-anthrachinon (I) 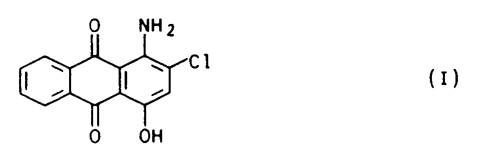 durch Chlorierung von 1-Amino-4-hydroxy-anthrachinon (II) 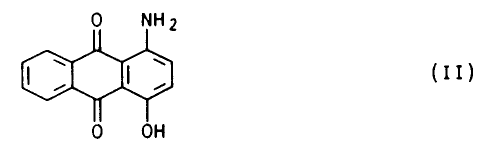 dadurch gekennzeichnet, daß man die Chlorierung in 90 bis 100 gew.-%iger Schwefelsäure oder in bis zu 2 gew.-%igem Oleum durchführt.

2. Verfahren gemäß Anspruch 1, dadurch gekennzeichnet, daß die Chlorierung bei 20 bis 100°C erfolgt.

## Claims

1. A process for the preparation of 1-amino-2-chloro-4-hydroxyanthraquinone (I) 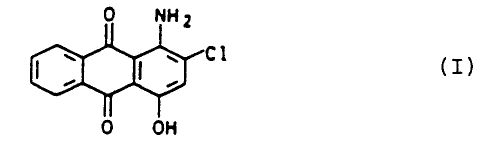 by chlorinating 1-amino-4-hydroxyanthraquinone (II) 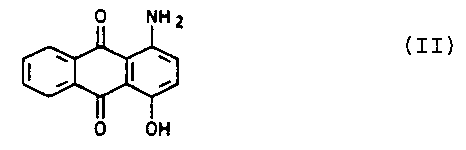 wherein the chlorination is carried out in 90-100% strength by weight sulfuric acid or in not more than 2% strength by weight oleum.

2. A process as claimed in claim 1, wherein the chlorination is carried out at from 20 to 100°C.

## Revendications

1. Procédé de préparation de la 1-amino-2-chloro-4-hydroxyanthraquinone (I) 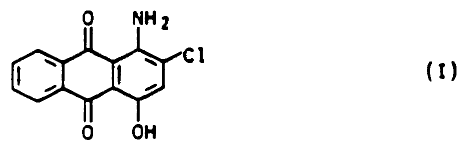 par chloration de la 1-amino-4-hydroxyanthraquinone (II) 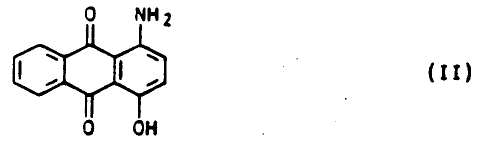 caractérisé en ce que l'on effectue la chloration dans de l'acide sulfurique à 90 à 100% en poids ou dans de l'oléum à une concentration pouvant aller jusqu'à 2% en poids.

2. Procédé selon la revendication 1, caractérisé en ce que la chloration est effectuée à une température comprise entre 20 et 100°C.
